# EUROPEAN PATENT SPECIFICATION

(11) **EP 1 554 583 B1**
(45) Date of publication and mention of the grant of the patent: **22.08.2007**
(21) Application number: 03758621.1
(22) Date of filing: 21.10.2003
(51) Int. Cl.: G01N 33/574, G01N 33/68

(54) **THE USE OF THE A3 ADENOSINE RECEPTOR AS A MARKER FOR A DISEASE STATE**
DIE VERWENDUNG VON DEM A3 ADENOSIN REZEPTOR ALS MARKER EINES KRANKHEITSZUSTANDES
UTILISATION DU RECEPTEUR DE L'ADENOSINE A3 EN TANT QUE MARQUEUR D'UN ETAT PATHOLOGIQUE

(30) Priority: 22.10.2002 US 420038 P
(43) Date of publication of application: 20.07.2005
(73) Proprietor: Can-Fite Biopharma Ltd., 49170 Petach Tikva (IL)
(72) Inventor: FISHMAN, Pnina, 46365 Herzliya (IL); MADI, Lea, 75791 Rishon Le Zion (IL); BAR YEHUDA, Sara, 75474 Rishon le Zion (IL)
(74) Representative: Colombet, Alain André
(86) International application number: PCT/IL2003/000856
(87) International publication number: WO 2004/038419

(56) References cited:
- WO-A-01/19360
- WO-A-97/38125
- US-B1- 6 376 521
- FISHMAN PNINA ET AL: "A3 adenosine receptor as a target for cancer therapy." ANTI-CANCER DRUGS. ENGLAND JUN 2002, vol. 13, no. 5, June 2002 (2002-06), pages 437-443, XP009024520 ISSN: 0959-4973
- MADI LEA ET AL: "High expression of A3ARS in melanoma and colon carcinoma cell lines: A target for tumor cell growth inhibition." DRUG DEVELOPMENT RESEARCH, vol. 56, no. 4, August 2002 (2002-08), page 560 XP009024523 Seventh International Symposium on Adenosine and Adenine Nucleotides;Queensland, Australia; May, 2002 ISSN: 0272-4391 (ISSN print)
- FISHMAN PNINA ET AL: "Pharmacology and therapeutic applications of A3 receptor subtype." CURRENT TOPICS IN MEDICINAL CHEMISTRY. NETHERLANDS 2003, vol. 3, no. 4, 2003, pages 463-469, XP009024514 ISSN: 1568-0266

## Description

### FIELD OF THE INVENTION

This invention relates to the field of diagnosis and in particular to biological markers associated with disease states, used for diagnosis.

### PRIOR ART

The following is a list of prior art which is considered to be pertinent for describing the state of the art in the field of the invention. Acknowledgement of these references herein will be made by indicating the number from their list below within brackets.
(1) Olah M.E. and Stiles G.L. The role of receptor structure in determining adenosine receptor activity, Pharmacol. There., 85:55-75 (2000);
(2) Poulsen S.A. and Quinn R.J.,Adenosine receptors: new opportunities for future drugs. Bioorg Med. Chem., 6:619-641 (1998);
(3) Fang X. et al. Phosphorylation and inactivation of glycogen synthase kinase 3 by protein kinase A., Proc. Natl. Acad. Sci. USA, 97:11960-11965 (2000);
(4) Fishman, P., et al., Involvement of Wnt Signaling Pathway in IB-MECA Mediated Suppression of Melanoma Cells, Oncogene 21:4060-4064 (2002);
(5) Ferkey, D.M., and Kimelman, D. GSK-3 : New Thoughts on an Old Enzyme, Dev Biol., 225:471-479 (2000);
(6) Bonvini, P., et al. Nuclear beta-catenin displays GSK-3beta- and APC-independent proteasome sensitivity in melanoma cells, Biochim. Biophys. Acta., 1495 :308-318 (2000);
(7) Olah, M.E. and Stiles, G.L, The role of receptor structure in determining adenosine receptor activity, Pharmacol. Ther., 85:55-75 (2000).

### BACKGROUND OF THE INVENTION

A₃ adenosine receptors belong to the family of the Gi-protein associated cell surface receptors. Receptor activation leads to its internalization and the subsequent inhibition of adenylate cyclase activity, cAMP formation and protein kinase A (PKA) expression, resulting in the initiation of various signaling pathways ^{(1,2)}. PKA contains a catalytic subunit PKAc which dissociates from the parent molecule upon activation with cAMP Recent studies have demonstrated that PKAc phosphorylates and inactivates a GSK-3β ⁽³⁾.

Recently, it has been shown that 1-deoxy-1-[6[[(3-iodophenyl)methyl] aminol-9H-purine-9-yl]-N-methyl-p-D-ribofura-nuronaminde (IB-MECA), a stable agonist to A3AR, alters the expression of GSK-3β and β-catenin, key components of the Wnt signaling pathway. Consequently it leads to inhibition of the expression of the cell cycle progression genes, c-myc and cyclin D1⁽⁴⁾.

Fishman et al. (2002) Anti-Cancer Drugs 13:437-443 report that targeting the A3 adenosine receptor by adenosine or a synthetic agonist of this receptor (IB-MECA and CI-IB-MECA) results in a differential effect on tumor and on normal cells.
Madi et al. (2001) Abstracts from Purines 72 report that high expression of A3ARs in melanoma and colon carcinoma cell lines defines a target for tumor cell growth inhibition.

### SUMMARY OF THE INVENTION

The present invention is based on the surprising finding that in cancer cells there is an increase in the level of A₃ adenosine receptor expression as compared to non-cancerous cells, obtained, for example, from the same subject from which the cancer cells were obtained. This finding paves the way for the use of the A₃ adenosine receptor expression level as a means for the diagnosis of a disease state.

Thus, according to one of its aspects, the present invention concerns a method of detecting a disease state consisting of a cancer or a tumor in a subject, comprising:
(a) obtaining from the subject a sample of cells suspected of being in a disease state;
(b) detecting the level of expression of A₃ adenosine receptor (A3AR) in said sample cells; and
(c) comparing the level of said A3AR expression in said cells to a control level, being the level of A3AR expression in normal cells of the subject, which normal cells belong to a same tissue as said sampled cells; wherein a difference in the level between the control and the sampled cells is indicative of said diseased state.

While the above method is qualitative or binary, i.e. indicates whether the person has the disease or not, the method may also be used, in some cases, in a quantitative manner, to assess the severity of the disease state, in a subject already diagnosed as having a disease, i.e. the larger the difference between the A₃ adenosine receptor expression level in the sampled cells suspected of having the diseased state, and the control level of expression A3AR, (being the expression level in normal cells, or the expression standard reference), the more severe is the disease

Thus by another aspect, the present invention concerns a method for determining the severity of a disease state in a subject comprising:
(a) obtaining from the subject a sample of cells suspected of being in a disease state;
(b) detecting the level of expression of A₃ adenosine receptor (A3AR) in said sampled cells; and
(c) comparing the level of A3AR expression in said cells with a predetermined calibration curve of the level of the A3AR; the values of the calibration curve being correlated to the severity of the disease state, thereby determining the severity of the disease state of the subject

By yet another aspect the present invention concerns utilizing the determination of A3AR expression level to try and predict whether a subject is suitable for therapeutic treatment by A3AR modulators, being A3AR agonists and A3AR antagonists, typically A3AR agonists. Example of such treatment is administration of an A3AR agonist such as IB-MECA or C1.IB.MECA, for the treatment of cancer or inflammatory diseases.

Where it is desired to predict, *a priori,* which patients have a better chance of response to treatment with A3AR modulators, in particular A3AR agonists, it is possible to use the above method to determine whether the cells, suspected of being in a disease state, express A3AR at a level significantly different from the control. In case of an affirmative answer, it may be predicted that the patient has a better probability to respond to a treatment with an A3AR modulator, especially an A3AR agonist.

Thus, the present invention concerns a method for determining whether a subject may be expected to respond to a therapeutic treatment of a disease state consisting of a cancer or a tumor by the administration of an A3AR agonist, the method comprising:
(a) obtaining from the subject a sample of cells associated with the disease state;
(b) detecting the level of expression of A₃ adenosine receptor (A3AR) in said sample; and
(c) comparing the level of said A3AR expression in said cells to a control level, said control level being the level of A3AR expression in normal cells of the subject, which normal cells belong to a same tissue as said sampled cells; wherein a difference between the control level and the level of the sampled cells is indicative that the subject has a high probability of responding to therapeutic treatment by the administration of A3AR agonists.

The term *"disease state"* as used herein refers to any disease state in which the A3AR-associated signal transduction pathways is known, or is experimentally found, to be involved. Such diseases, according to the invention, may be associated with an abnormal and undesired rate of cell proliferation so that the treatment of the disease requires modulation (either an increase or a decrease) of the cell cycle. Proliferative diseases characterized by excess proliferation include, without being limited thereto, all types of cancer; and in particular all types of solid tumors; skin proliferative diseases, and disease characterized by excessive formation of blood vessels such as restinosis. Degenerative diseases characterized by undesired death of cells include neurodegenerative or neurotraumatic diseases ; such as Alzheimer's disease, frontal lobe degeneration, argyrophilic grains disease or sebacute scleroting panecephalitis; neurotraumatic diseases such as acute stroke, schizophrenia, or manic depression; autoimmune diseases (including rheumatoid arthritis (RA), Crohn's Disease (CD) and multiple sclerosis (MS); non-insulin dependent diabetes mellitus (insulin type II diabetes), and others.

By a preferable embodiment the disease is a tumor, preferably solid tumors.

The term "*solid tumors"* refers to carcinomas, sarcomas, adenomas, and cancers of neuronal origin and in fact to any type of cancer which does not originate from hematopoeitic cells, and in particular concerns: carcinoma, sarcoma, adenoma, hepatocellular carcinoma, hepatocellularcarcinoma, hepatoblastoma, rhabdomyosarcoma, esophageal carcinoma, thyroid carcinoma, ganglioblastoma, fibrosarcoma, myxosarcoma, liposarcoma, cohndrosarcoma, osteogenic sarcoma, chordoma, angiosarcoma, endotheliosarcoma, lymphagiosarcoma, synovioama, Ewing's tumor, leimyosarcoma, rhabdotheliosarcoma, colon carcinoma, pancreatic cancer, breast cancer, ovarian cancer, prostate cancer, squamous cell carcinoma, basal cell carcinoma, adenocarcinoma, renal cell carcinoma, hematoma, bile duct carcinoma, melanoma, choriocarcinoma, seminoma, embryonal carcinoma, Wilms' tumor, cervical cancer, testicular tumor, lung carcinoma, small lung carcinoma, bladder carcinoma, epithelial carcinoma, glioma, astrocyoma, medulloblastoma, craniopharyngioma, ependynoma, pinealoma, rotinoblastoma, multiple myeloma, rectal carcinoma, thyroid cancer, head and neck cancer, brain cancer, cancer of the peripherial nervous system, cancer of the central nervous system, neuroblastoma, cancer of the edometrium, as well as metastasis of all of the above. It has been shown in accordance with the invention that increased expression of A3AR can be found not only in the primary tumor site but also in metastasis thereof.

The term *"cells suspected of being in a disease state"* refers to cells, tissue samples or cell components (such as cellular membranes or cellular components) which are suspected of manifesting the disease. For example, where the disease is cancer of type X, the cells are the cells of the tissue of the cancer (breast, colon, skin, liver, lungs, cells, etc.) suspected of being transformed. Where the disease is cancer the cells suspected of being transformed may be obtained by methods known for obtaining *"suspicious"* cells such as by biopsy, needle biopsy, etc. The suspicion of being in a disease state, may be raised due to various imaging (NMR, MR, scanning, ultrasound, memographic), pathological or histological techniques.

Where, for example, the disease is psoriasis, the cells are skin cells. Where, by another example, the disease is an autoimmune disease, the cells may be cells of the immune system or tissue attacked by the immune system (synovium in the case of RA, nerve cells in MS, etc.) etc.

The cells may be obtained by means well known in the art such as, for example, by drawing blood, by tissue biopsy, needle biopsy, tissue aspiration, etc.

The term *"A3AR* ", refers to adenosine A3 receptor (protein) and a fragment thereof, which may be for example, an extracellular fragment present on the external surface of the cell, as well as to mRNA of the A3AR receptor or a fragment of said mRNA.

The term *"detecting the level of A3AR in said cells"* refers to any technique known in the art to detect the presence of a protein, or a fragment of a protein in cells either in the cytosol, the membrane, or in any intracellular component of the cells, as well as to techniques for the detection of mRNA levels (including fragments of the full mRNA) in any component of the cells.

Methods for detecting the level of the protein may include: extracting the protein contents of the cells, or extracting fragments of protein from the membranes of the cells, or from the cytosol, for example, by using state of the art lysis, digestive, separation, fractionation and purification techniques, and separating the proteinaceous content of the cells (either the crude contents or the purified contents) on a Western Blot, and then detecting the presence of the A3AR protein, or A3AR protein fragment by various identification techniques known in the art. For example the contents separated on a gel may be identified by using suitable molecular weight markers together with a protein identification technique, or using suitable detecting moieties (such as labeled antibodies, labeled lecithins, labeled agonists or antagonists to the A3AR receptor) attached to a labeled moiety. The detection may also be by *in situ* binding of specific recognition agents, to A3AR either when present *"on the cells", in situ,* or even when present on cells in the tissue and such agents may be labeled A3AR agonists, such as labeled IB-MECA ; labeled A3AR antagonists MG132, antibodies against A3AR such as those mentioned in the detailed examples, etc., and then detecting the presence of the recognition moieties using techniques suitable for the nature of the marker.

Where the recognition agents are fluorescent-labeled the detection may be carried out by using a confocal microscope and directly viewing the level of labeled moieties bound to the receptor. Where the recognition agents are radio-labeled, the level may be determined by the determination of the radio-label level in the cells.

The determination of the A3AR expression level may also comprise determination of the mRNA level (or a fragment of the full mRNA). For example, the detection may be by any methods used in the art for the detection of RNA in a cell-containing sample such as by *using in situ* hybridization with a detectable probe, for example, with a complementary sequence containing a detectable moiety (fluorescent, radioactive, chromatophoric moiety, etc). In such a case of *in situ* hybridization there is no need to extract the RNA from the cells and all that is needed is treatment to render the cells porous. However various amplification methods, which are sensitive enough to detect minute amounts of RNA are preferable. Such methods include, PCR, RT-PCP, *in situ* PCR, *in situ* RT-PCR (all of the above referring also to *"nested"* PCR, and nested RT-PCR), LCR (ligase chain reaction) and 3SR (self sustained sequence replication). In accordance with a preferred embodiment, RT-PCR and nested RT-PCR are used. The amplification products are identified by methods used in the art such as by separation on a gel and detection using a suitable labeled probe.

The sample may be cell membranes, proteins extracted from cell membranes, whole cells, cytosolic contents of cells, tissue samples obtained from a subject including paraffin embedded tissue samples, proteins obtained from the cytosol or mRNA obtained from the nucleus or cytosol.

The level of A3AR expression in the diseased-state cells should be compared to the control expression level. This *control level* may be defined as the level of the same molecule (e.g. protein, protein fragment) obtained from the same cellular component in non-diseased cells obtained from the same subject, preferably from the same tissue from which the cells suspected of being in a disease state were obtained. For example, if the diseased cells are breast cancer cells obtained from a suspected tumor site (suspected, for example, due to imaging techniques), the control may be the level of A3AR expression in breast cells obtained from a site other than the tumor site, preferably from cells obtained adjacent to the tumor site. Alternatively, the control A3AR level may be obtained from a normal control, for example, by determining the A3AR expression level in a pool of cells (of the same type as the diseased cells) obtained from a plurality of normal subjects.

The term *"a difference in the level"* may refer to any statistically significant difference. Alternatively, a 'threshold difference' may be determined by determining the difference between the average level in a plurality of diagnosed diseased samples and the average level in a plurality of diagnosed non-diseased control samples. A difference larger than the threshold difference will be considered as a *"difference in the level"* between treatment and control and a difference smaller than said threshold difference will not be considered as a *"difference in the level".*

In accordance with the invention, any difference (statistically significant, or by using a threshold difference as described above) between the level of expression of A3AR in the suspected diseased cells, as compared to control cells (either non-diseased cells obtained from the same subject, or from standard healthy controls) is indicative of the existence of the diseased state. Typically, in accordance with the invention, an increase in the level of A3AR as compared to control is indicative of the presence of a proliferative disease such as cancer, other proliferative diseases such as psoriasis or angiogenic related diseases (such as restinosis), inflammatory diseases such as RA, CD or MS.

At times, it is desired to determine the severity of the disease, especially in patients that have already been positively diagnosed as having the disease. In such cases, the level of A3AR expression should be quantified, by comparison to a calibration curve prepared beforehand. Such calibration curve is prepared by determining the level of A3AR expression (which may be the level of A3AR protein, protein fragment, or mRNA level etc., as discussed above) present in cells obtained from a plurality of patients positively diagnosed (by other means, for example by a physician, by histological techniques etc.) as having the diseased state at varying levels of severity of the disease, as a function of the severity of the disease, for example, as a function of the grade of the disease, the tumor mass, appearance of metastasis, number of metastasis, mortality (from slides stored for a period after they were obtained). The severity of the disease for the preparation of the calibration curve may also be determined by various acceptable methods such as by pathological techniques.

For example, a protein content level of between X₁ to X₂ per 1,000,000 cells may be defined as being indicative of grade 1 cancer, a higher protein content of Y₁ to Y₂ per 1,000,000 cells may be defined as being indicative of grade 2 cancer, etc. The calibration curve may plot the expression level as a function of the grade of the disease, the tumor size, whether the disease is a primary or metastatic tumor etc. After such a calibration curve is prepared, it is possible to compare the level of A3AR expression obtained from a specific individual to the corresponding value in the calibration curve, and thus obtain a certain assessment of the severity of the disease.

In accordance with the present invention, it is possible by using the method of the invention to detect cancer together with additional tumor markers, preferably together with tissue specific tumor markers, in order to increase the sensitivity and decrease false-positive/false-negative results.

Examples of such tumor markers are: CEA, CK19, CK20, c-Met, MAGE-A3, b-hCG, GalNAc-T, CK18, Mucin-1 (MUC-1), and carcinoembryonic antigen (for breasr and colon); EWS-FL11EWS (for Ewing sarcoma, pNET's); ERG,PAX3-FKHR,FAX7-FKHR (for alveolair rhabdomyo- sarcoma); prostate specific antigen (PSA), prostate membrane specific antigen (prostate cancer); tyrosine hydroxylase, PGP 9.5 (for neuroblastoma), tyrosinase, PG6 9.5. MAGE (for melanoma), alpha-fetoprotein, albumin (for hepatoma); cytokeratins (epithelial cells).

### BRIEF DESCRIPTION OF THE DRAWINGS

In order to understand the invention and to see how it may be carried out in practice, some preferred embodiments will now be described, by way of nonlimiting examples only, with reference to the accompanying drawings, in which:
**Fig. 1A** shows immunohistochemistry of normal and tumor tissue from a carcinoma lesion.
**Fig. 1B** shows a Western Blot of A3AR obtained from human carcinoma cells and adjacent normal tissue.
**Fig. 2** shows a Western Blot of A3AR obtained from three patients having colon cancer compared to A3AR levels in adjacent normal tissue.
**Fig. 3** shows a Western Blot of A3AR obtained from breast tumor cells (right) as compared to the level in normal adjacent breast cells (left).
**Fig. 4A** shows a paraffin embedded histological section obtained by needle biopsy of the subject tested in Fig 4B.
**Fig. 4B** shows separation of RT-PCR products of mRNA amplification obtained from normal breast cells of a patient and from breast tumor cells, A3AR was identified by molecular weight marker.
**Fig. 5** shows Western (top) or Northern (bottom) Blots of A3AR expression from a colon and breast patient from normal and tumor tissue.
**Fig. 6** shows RT-PCR of A3AR mRNA obtained from normal and tumor cells from primary melanoma (right) and metastasic melanoma.

### EXPERIMENTAL PROCEDURES

### Materials and Methods

IB-MECA and MRS 1523 were purchased from RBI/Sigma (Natick, MA, USA). For both reagents, a stock solution of 10 mM was prepared in DMSO and further dilutions in RPMI medium were performed.

RPMI, fetal bovine serum (FBS) and antibiotics for cell cultures were obtained from Beit Haemek, Haifa, Israel.

Rabbit polyclonal antibodies against murine and human A3AR, were purchased from Santa Cruz Biotechnology Inc., Ca, USA.

Rabbit polyclonal antibodies against murine and human cyclin D 1 (Upstate, NY), A_{2B} adenosine receptor, Cy3-conjugated anti-goat IgG and Fluorescein-conjugated anti-rabbit IgG were purchased from Chemicon, Ca.

### Identification of A3AR Protein

### Western Blot Analysis

To detect the level of expression of the A3AR proteins Western blot analysis was performed. Cells were transferred to ice-cold lysis buffer (TNN buffer, 50mM Tris buffer pH=7.5, 150mM NaCl, NP 40). Cell debris was removed by centrifugation for 10 min, at 7500xg. The supernatant was utilized for Western Blot analysis. Protein concentrations were determined using the Bio-Rad protein assay dye reagent. Equal amounts of the sample (50µg) were separated by SDS-PAGE, using 12% polyacrylamide gels. The resolved proteins were then electroblotted onto nitrocellulose membranes (Schleicher & Schuell, Keene, NH, USA). Membranes were blocked with 1% bovine serum albumin and incubated with the A3AR primary antibody (dilution 1:1000) for 24h hour at 4°C. Blots were then washed and incubated with a secondary antibody for 1h at room temperature. Bands were recorded using BCIP/NBT color development kit (Promega, Madison, W1, USA). Data presented in the different figures are representative of at least three different experiments.

### Identification of A3AR mRNA

### Northern Blot Analysis

Total RNA was -from cells, utilizing Tri-reagent (Sigma, Saint -Louis). The samples were then subjected twice to phenol:chloroform extraction and washed with chloroform. RNA was precipitated with sodium acetate/ethanol following washing with ethanol, then denatured, separated (25 µg per lane) in 1.1% formaldehyde agarose gels and transferred to Hybond-N membrane. The 390 bp *EcoR*I fragment from A3AR cDNA clone of mouse (TAA3I.S), kindly supplied by Dr Kathia Ravid, was prepared by random-primed synthesis. Probes were used in RNA blot analysis at a hybridization temperature of 42°C in the presence of 50% formamide.

### Example 1: Determination of A3AR protein expression level in colon cells vs. normal cells

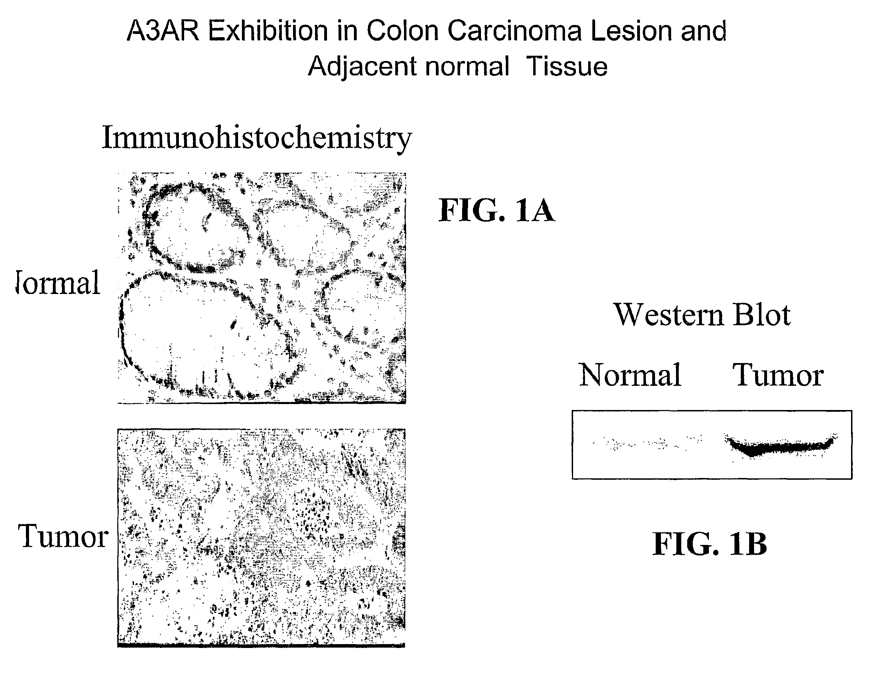

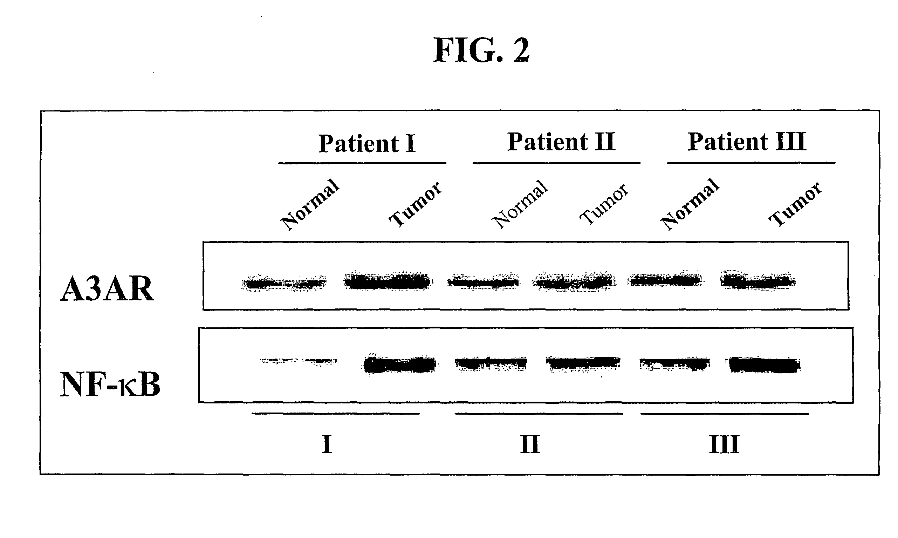

Samples were obtained from colon carcinoma and breast cancer patients during biopsy. Fig. 1A shows an immunohistochemistry slide of a cancer patient
wherein sections were identified by a pathologist as being normal (top) or cancerous (bottom). Cells obtained from the consecutive section shown in Fig 1A had their contents extracted and the A3AR underwent Western blot separation and A3AR was identified using A3AR antibodies and labeled anti-rabbit IgG antibodies. As can be seen from Fig. 1B, tumor cells showed a significantly higher level of A3AR protein expression than normal cells obtained from the same subject. Fig 2 shows the same results for three different colon cancer patients and as can be seen, in all three cases the level of A3AR expression was higher in the cancer cells of the patient as compared to the normal cells.

### Example 2: Determination of A3AR protein and expression level in breast cancer cells vs. normal cells

Breast cancer cells identified by a pathologist were obtained by biopsy from the subject together with normal breast cancer cells from the adjacent tissue. The protein contents of the two types were identified by separation on a Western Blot and using labeled antibodies.

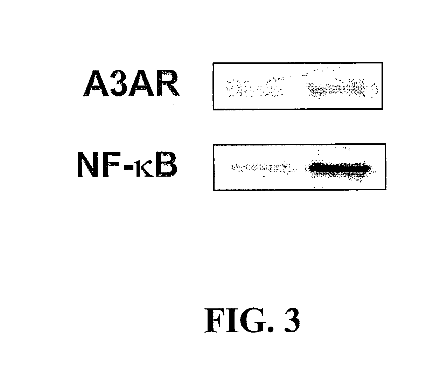

The results are shown in Fig. 3. As can be seen, breast tumor cells expressed a significantly higher level of the A3AR protein expression as compared to the adjacent normal cells.

### Example 3: Determination of A3AR mRNA expression level in breast cancer patients vs. normal cells

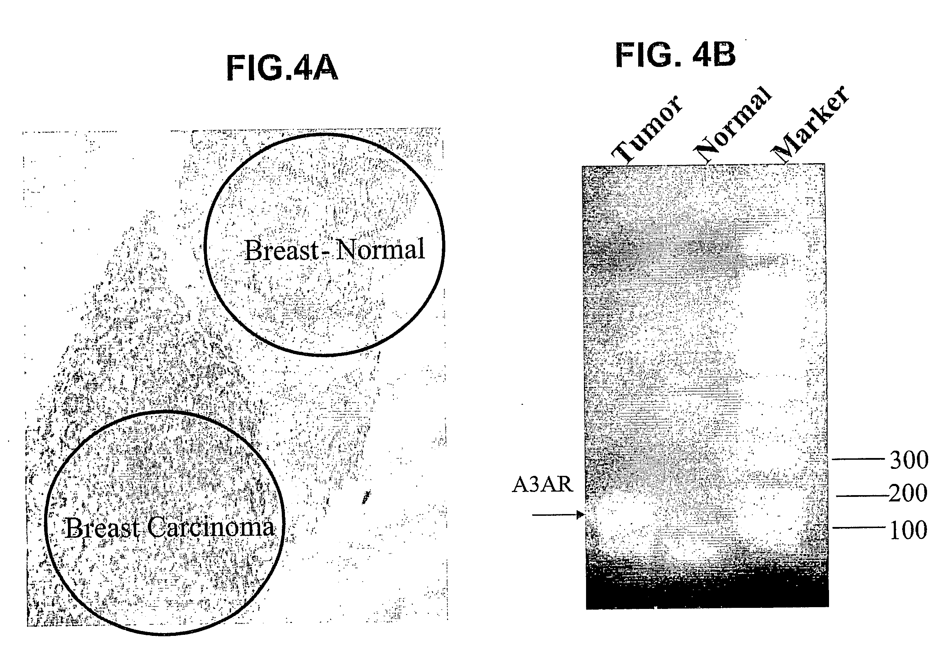

A sample from a region suspected as being cancerous in the breast of a patient was obtained using needle biopsy. The sample was sent to a pathologist who marked normal and breast carcinoma cells (Fig. 4A). RT-PCR was performed on the tumor cells and normal cells obtained from the consecutive section to that marked in Fig. 4A, and the amplification products were separated by Northern blot and identified using suitable molecular weight markers. The results are shown in Fig. 4B. As can be seen, the A3AR mRNA levels were significantly higher in breast tumor cells as compared to normal cells. This indicates that cancer can be detected by using both mRNA and protein determination of expression levels.

### Example 4: Simultaneous detection of A3AR protein and mRNA level in samples obtained from colon cancer and breast cancer patients

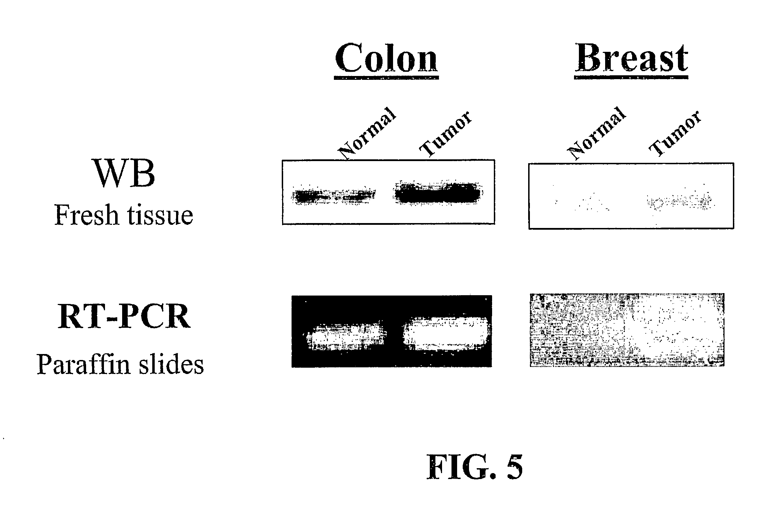

Normal and cancerous cells obtained from colon and breast cancer patients were identified by a pathologist and cell samples were obtained from each tissue. The A3AR protein level was determined from the tumor and cancerous cells by using a Western Blot followed by identification with antibodies, and the RNA level was determined by using RT-PCR amplification followed by separation on a Northern Blot. The results are shown in Fig. 5. As can be seen, a cancerous state of both breast and colon cancer was determined by detecting a higher level of A3AR protein expression or A3AR mRNA expression in the cancer cells as compared to the level in the non-cancer cells.

### Example 5 : Detection of A3AR mRNA level in primary and metastasic melanoma

Cells identified by a pathologist as melanoma cells were obtained from a primary melanoma site, and cancerous cells were also obtained from a secondary melanoma site. In addition cells identified by the pathologist as normal were obtained from the same patient.

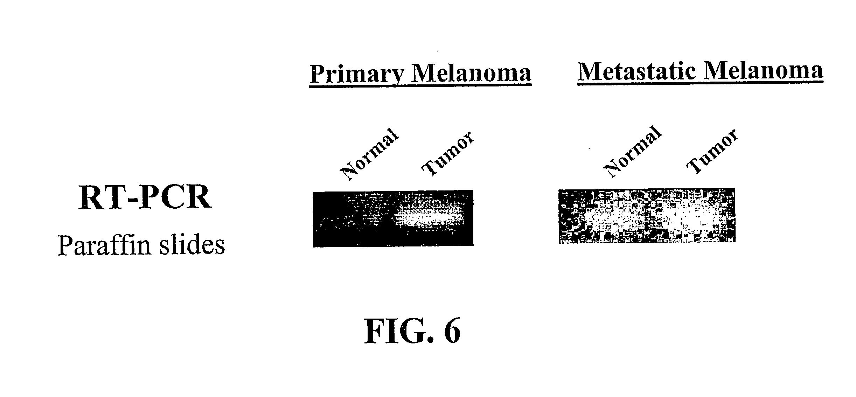

The mRNA contents of the melanoma primary cells, metastasic cells and normal cells were amplified using RT-PCR and separated on a northern blot. The results are shown in Fig. 6. As can be seen, both primary and metastasic tumors expressed a higher level of A3AR mRNA than normal cells, indicating that the method of the invention is also suitable for the detection of metastasic cancer.

## Claims

1. A method of detecting a disease state consisting of a cancer or a tumor in a subject, comprising:
(a) detecting the level of expression of A₃ adenosine receptor (A3AR) in a sample of cells obtained from the subject and suspected of being in the disease state; and
(b) comparing the level of said A3AR expression in said sampled cells to the level of A3AR expression in normal cells of the same subject, which normal cells belong to a same tissue as said sampled cells, wherein a difference in the level between the normal and the sampled cells is indicative of said diseased state.

2. The method of Claim 1, wherein the difference is an increase in the level of the A3AR expression level as compared to the normal cells.

3. The method of Claim 1 or 2, wherein the tumor is a solid tumor.

4. A method for determining the severity of a disease state consisting of a cancer or a tumor in a subject comprising:
(a) detecting the state of expression of A₃ adenosine receptor (A3AR) in a sample of cells obtained from the subject and suspected of being in the disease state; and
(b) comparing the level of A3AR expression in said cells with a predetermined calibration curve of the level of the A3AR ; the values of the calibration curve being correlated to the severity of the disease state, thereby determining the severity of the disease state of the subject.

5. A method according to Claim 4, wherein the tumor is a solid tumor.

6. A method according to any of Claims 1-5, wherein the A3AR expression level is determined by detecting the level of A3AR protein, or A3AR protein fragment in the sampled cells.

7. A method according to any of Claims 1-5, wherein the A3AR expression level is determined by detecting the level of A3AR mRNA in the sampled cells.

8. A method for determining whether a subject has a high probability of responding to a therapeutic treatment of a disease state consisting of a cancer or a tumor by the administration of an A3AR agonist or an A3AR antagonist , the method comprising:
(a) detecting the level of expression of A₃ adenosine receptor (A3AR) in a sample of cells obtained from the subject and associated with the disease state; and
(b) comparing the level of said A3AR expression in said sampled cells to the level of A3AR expression in normal cells of the subject, which normal cells belong to a same tissue as said sampled cells, wherein a difference in the level between the normal and the sampled cells is indicative that the subject has a high probability of responding to a therapeutic treatment by an A3AR agonist or A3AR antagonist.

9. A method according to Claim 8, wherein the difference in the level is an increase in the level of A3AR expression in the sampled cells as compared to normal cells.

## Patentansprüche

1. Verfahren zur Detektierung eines Erkrankungszustandes, bestehend aus Krebs oder einem Tumor in einem Patienten, umfassend:
(a) Detektieren des Expressionsanteils an A₃-Adenosin-Rezeptor (A3AR) in eine Zellprobe, die von einem Patienten erhalten wurde, der unter dem Verdacht des Erkrankungszustands steht; und
(b) Vergleichen des Anteils an A3AR-Expression in der Zellprobe mit dem Anteil an A3AR-Expression in normalen Zellen desselben Patienten, wobei die normalen Zellen zu demselben Gewebe wie die Zellproben gehören, wobei ein Unterschied in dem Anteil zwischen den normalen und den Zellproben ein Anzeichen für den Erkrankungszustand darstellt.

2. Verfahren nach Anspruch 1, worin der Unterschied ein Anstieg in dem Anteil an A3AR-Expression im Vergleich zu dem von normalen Zellen darstellt.

3. Verfahren nach Anspruch 1 oder 2, worin der Tumor ein fester Tumor ist.

4. Verfahren zur Herstellung der Schwere eines Erkrankungszustandes, bestehend aus Krebs oder einem Tumor in einem Patienten, umfassend:
(a) Detektieren des Expressionsanteils an A₃-Adenosin-Rezeptor (A3AR) in eine Zellprobe, die von einem Patienten erhalten wurde, der unter dem Verdacht des Erkrankungszustands steht; und
(b) Vergleichen des Anteils an A3AR-Expression in den Zellen mit einer vorbestimmten Kalibrierungskurve des Anteils an A3AR; wobei die Werte der Kalibrierungskurve in Korrelation zu der Schwere des Erkrankungszustands stehen, wodurch die Schwere des Erkrankungszustands des Patienten bestimmt wird.

5. Verfahren nach Anspruch 4, worin der Tumor ein fester Tumor ist.

6. Verfahren nach einem der Ansprüche 1 bis 5, worin der Anteil an A3AR-Expression durch Detektieren des Anteils an A3AR-Protein oder A3AR-Proteinfragment in den Zellproben bestimmt wird.

7. Verfahren nach einem der Ansprüche 1 bis 5, worin der Anteil an A3AR-Expression durch Detektieren des Anteils an A3AR-mRNA in den Zellproben bestimmt wird.

8. Verfahren zur Bestimmung, ob ein Patient eine hohe Wahrscheinlichkeit der Reaktion auf eine therapeutische Behandlung eines Erkrankungszustands, bestehend aus Krebs oder einem Tumor, durch Verabreichung eines A3AR-Agonisten oder eines A3AR-Antagonisten aufweist, wobei das Verfahren umfasst:
(a) Detektieren des Expressionsanteils an A₃-Adenosin-Rezeptor (A3AR) in eine Zellprobe, die von einem Patienten erhalten wurde, der unter dem Verdacht des Erkrankungszustands steht; und
(b) Vergleichen des Anteils an A3AR-Expression in der Zellprobe mit dem Anteil an A3AR-Expression in normalen Zellen desselben Patienten, wobei die normalen Zellen zu demselben Gewebe wie die Zellproben gehören, wobei ein Unterschied in dem Anteil zwischen den normalen und den Zellproben ein Anzeichen dafür ist, dass der Patient eine hohe Wahrscheinlichkeit einer Reaktion gegenüber einer therapeutischen Behandlung durch einen A3AR-Agonisten oder A3AR-Antagonisten aufweist.

9. Verfahren nach Anspruch 8, worin der Unterschied in dem Anteil ein Anstieg in dem Anteil von A3AR-Expression in den Zellproben im Vergleich zu normalen Zellen darstellt.

## Revendications

1. Procédé de détection d'un état pathologique consistant en un cancer ou une tumeur chez un individu, comprenant :
(a) la détection du niveau d'expression du récepteur de l'adénosine A₃ (A3AR) dans un échantillon de cellules obtenues chez l'individu et suspectées d'être dans l'état pathologique ; et
(b) la comparaison du niveau de ladite expression de A3AR dans lesdites cellules d'échantillon avec le niveau d'expression de A3AR dans des cellules normales du même individu, lesquelles cellules normales appartiennent à un même tissu que lesdites cellules d'échantillon, où une différence de niveau entre les cellules normales et les cellules d'échantillon est révélatrice dudit état pathologique.

2. Procédé selon la revendication 1, dans lequel la différence est une augmentation du niveau d'expression de A3AR comparé aux cellules normales.

3. Procédé selon la revendication 1 ou 2, dans lequel la tumeur est une tumeur solide.

4. Procédé pour déterminer la gravité d'un état pathologique consistant en un cancer ou une tumeur chez un individu comprenant :
(a) la détection de l'état d'expression du récepteur de l'adénosine A₃ (A3AR) dans un échantillon de cellules obtenues chez l'individu et suspectées d'être dans l'état pathologique ; et
(b) la comparaison du niveau d'expression de A3AR dans lesdites cellules avec une courbe d'étalonnage prédéterminée du niveau de A3AR ; les valeurs de la courbe d'étalonnage étant corrélées à la gravité de l'état pathologique, en déterminant ainsi la gravité de l'état pathologique de l'individu.

5. Procédé selon la revendication 4, dans lequel la tumeur est une tumeur solide.

6. Procédé selon l'une quelconque des revendications 1 à 5, dans lequel le niveau d'expression de A3AR est déterminé par la détection du niveau de la protéine A3AR, ou d'un fragment de protéine A3AR, dans les cellules d'échantillon.

7. Procédé selon l'une quelconque des revendications 1 à 5, dans lequel le niveau d'expression de A3AR est déterminé par la détection du niveau de l'ARNm de A3AR dans les cellules d'échantillon.

8. Procédé pour déterminer si un individu présente une forte probabilité de réponse à un traitement thérapeutique d'un état pathologique consistant en un cancer ou une tumeur par l'administration d'un agoniste de A3AR ou un antagoniste de A3AR, le procédé comprenant:
(a) la détection du niveau d'expression du récepteur de l'adénosine A₃ (A3AR) dans un échantillon de cellules obtenues chez l'individu et associées à l'état pathologique ; et
(b) la comparaison du niveau de ladite expression de A3AR dans lesdites cellules d'échantillon avec le niveau d'expression de A3AR dans des cellules normales de l'individu, lesquelles cellules normales appartiennent à un même tissu que lesdites cellules d'échantillon, où une différence de niveau entre les cellules normales et les cellules d'échantillon est révélatrice que l'individu présente une forte probabilité de réponse à un traitement thérapeutique par un agoniste de A3AR ou un antagoniste de A3AR.

9. Procédé selon la revendication 8, dans lequel la différence de niveau est une augmentation du niveau de l'expression de A3AR dans les cellules d'échantillon comparée aux cellules normales.
